# EUROPEAN PATENT SPECIFICATION

(11) **EP 0 894 494 B1**
(45) Date of publication and mention of the grant of the patent: **16.04.2003**
(21) Application number: 98305982.5
(22) Date of filing: 28.07.1998
(51) Int. Cl.: A61K 7/48

(54) **Cell renewal rate compositions**
Zusammensetzungen für die Beschleunigung der Zellerneuerung
Compositions accélérant le renouvellement de cellules

(30) Priority: 29.07.1997 US 902084; 14.04.1998 US 60435
(43) Date of publication of application: 03.02.1999
(73) Proprietor: AMWAY CORPORATION, Ada, Michigan 49355 (US)
(72) Inventor: Zimmerman, Amy C., Grand Rapids, Michigan 49505 (US); Beio, Daniel, Ada, Michigan 49301 (US)
(74) Representative: Baverstock, Michael George Douglas

(56) References cited:
- EP-A- 0 676 194
- DE-C- 19 515 609
- FR-A- 2 597 337
- FR-A- 2 720 643
- US-A- 5 470 874

## Description

### BACKGROUND OF THE INVENTION

The present invention relates to a composition to enhance the rate of skin cell renewal or exfoliation and to a method of increasing the skin cell renewal rate. In particular, the present invention relates to a composition containing hydroxycarboxylic acid and an enhancing effective amount of acerola cherry fermentate or oat extract, or both. The present invention also relates to a method of increasing the rate of skin-cell renewal by applying a composition to the skin, wherein the composition comprises a hydroxycarboxylic acid and an enhancing effective amount of acerola cherry fermentate or oat extract, or both.

With the aging population, there is a continuing effort to provide cosmetic compositions to improve the appearance of the skin. Thus, many compositions are directed to improving the moisture retaining properties of the skin and consequently, include known moisturizers. At the same time, many consumers seek to use only natural ingredients. As a result, moisturizers such as oatmeal and related oat products have been incorporated into cosmetic compositions.

Recently, in addition to improving the moisture retaining properties of the skin, many compositions have included hydroxycarboxylic acids such as glycolic, lactic, citric, and malic acids in an effort to increase the exfoliation or desquamation of the outermost layer of skin. Thus, cosmetic compositions containing hydroxycarboxylic acids are being marketed for such uses as dry skin, the reduction of wrinkles and fine lines, and to combat the effects of aging.

Human skin may be classified into two major parts: the outer layer or epidermis and an underlying layer or dermis. The dermis contains, among other things, blood vessels, nerves, collagen, elastin, and fibroblast cells, which are responsible for the biosynthesis of collagen and elastin.

The epidermis itself also may be considered to consist of two major zones, an inner or Malpighian layer and an outer or horny layer. The Malpighian layer, a living tissue, may be further divided into basal, spinous, and granular layers. The homy layer, a dead tissue, is also referred to as *stratum comeum.*

In the natural process, basal cells in the basal layer move outward through the spinous and granular layers to become dead cells called corneocytes, in the stratum corneum. The stratum corneum consists of approximately 14 layers of corneocytes. In the normal skin it takes about 14 days for the basal cells to move from the basal layer to the end of the granular layer and to become corneocytes, and another 14 days to reach the outermost layer of the stratum corneum, where they are naturally shed or exfoliated. This process of forming corneocytes is called keratinization, and stratum corneum are the natural products produced by this process. The stratum corneum is the skin tissue that one feels when touching the skin. Usually, it takes about 28 days for cells of the basal layer to move outward to the surface in the course of making new skin.

It is thought that by increasing the natural desquamation rate of the outermost part of the stratum corneum and thus exposing lower layers of the stratum corneum, the appearance of the skin will be improved. Many substances are known to increase the rate of natural desquamation but recently hydroxycarboxylic acids have received an increasing amount of attention. A drawback to the use of hydroxycarboxylic acids is that they can irritate the skin of the user. Thus, it would be desirable to incorporate a substance to reduce the possible irritating effect of the hydroxycarboxylic acid. It would also be desirable to increase the rate of desquamation beyond that provided by the hydroxycarboxylic acid without further increasing the possibility of skin irritation.

FR-A-2 720 643 describes a cosmetic preparation comprising free hydroxylated alpha acid, neutralized alpha acid and hydroxylated alpha acid chemically connected with a protein. The specific disclosure is that the hydroxylated alpha acid which is chemically connected with a protein is malic acid and the combination is oat protein mallate.

DE 195 15 609 describes a composition for the treatment of hair and scalp, particularly for the treatment of itching. The composition comprises an aqueous-alcoholic or alcoholic extract of oat seed (Avena sativa), particularly oatmeal, preferably in combination with hydroxycarboxylic acids.

### SUMMARY OF THE INVENTION

In accordance with the present invention, there is provided a composition for topical use comprising an organic acid (such as hydroxycarboxylic acid), and either acerola cherry fermentate or oat extract, or both. Preferably, the composition includes both the acerola cherry fermentate and the oat extract. When present, the acerola cherry fermentate is present in an amount effective to enhance the skin cell exfoliation rate of the organic acid. It is believed that the acerola cherry fermentate synergistically enhances the rate of skin exfoliation when combined with an organic acid, wherein the organic acid is preferably chosen from hydroxycarboxylic acids, keto carboxylic acids, and mixtures thereof. When present, the oat extract is present in an amount effective to enhance the skin cell desquamation rate of the hydroxycarboxylic acid. It is believed that the oat extract synergistically enhances the rate of skin desquamation when combined with a hydroxycarboxylic acid and, at the same time, reduces the potential for skin irritation from the hydroxycarboxylic acid.

In accordance with this aspect of the present invention, there is provided a composition comprising a hydroxycarboxylic acid, and either acerola cherry fermentate or oat extract, or both wherein each, if present, is present in a therapeutically effective amount in topically acceptable vehicle for application to human skin to enhance the rate of skin desquamation provided by the hydroxycarboxylic acid. Surprisingly, the enhancement in the rate of skin desquamation provided by the acerola cherry fermentate and/or the oat extract does not cause additional skin irritation. To the contrary, the incorporation of the oat extract reduces the potential for skin irritation caused by the hydroxycarboxylic acid.

Preferably, the composition can be used for the topical treatment and/or alleviation of dry skin and other skin maladies.

Although it is known that the acerola cherry fermentate contains a minor amount of organic acids and a minor amount of carboxylic acids, typically they are present in an amount no greater than about 5% by weight, preferably no greater than about 3%, and more preferably, no greater than about 2% of the acerola cherry fermentate. The acids include lactic, citric, malic, tartaric, ascorbic acids with lactic comprising about 92% by weight of all the acids. Surprisingly, it has been found that when the acerola cherry fermentate is combined with an organic acid, the exfoliation rate is increased beyond that which would be expected from the additional minor amount of acids found in the acerola cherry fermentate.

The terms "minor amount of organic acids" and "minor amount of carboxylic acids" as used in the specification and accompanying claims means that any organic acid or carboxylic acid, which includes saturated and unsaturated carboxylic and dicarboxylic acids, hydroxymonocarboxylicic acids, hydroxydicarboxylic acids, and hydroxytricarboxylic acids, is present in an amount no greater than about 5% by weight, preferably no greater than about 3%, and more preferably, no greater than about 2% of the acerola cherry fermentate.

In the composition according to the present invention, the amount of acerola cherry fermentate to be used can not be absolutely specified because it varies according to the form of preparation. However, it is generally used in an amount from about 0.01% to about 50%, more generally from about 0.1% to about 10%. Preferably the acerola cherry fermentate is used in an amount from about 0.5% to about 8%, more preferably from about 1% to about 5% based on the whole weight of the composition.

The composition also contains from about 0.01% to about 99% of a hydroxycarboxylic acid and may contain from about 0.01% to about 99% of oat extract in the absence of or in conjunction with acerola cherry fermentate.. The oat extract is derived from oat, *avena sativa.* Preferably, the oat extract does not contain β-glucan. In other words, the oat extract does not contain a measurable amount of β-glucan, e.g., less than about 0.01%. The oat extract may be incorporated into a solvent for ease of handling. For example, in a preferred embodiment, the oat extract is Incorporated in a 1:1 v/v mixture of 1,3 butylene glycol and water.

Another aspect of the present invention includes a method of increasing the rate of skin exfoliation or desquamation comprising topically applying a composition containing an organic acid, and either an enhancing effective amount of acerola cherry fermentate or an enhancing effective amount of oat extract, or both. In this aspect, the method includes topically applying to the skin a composition comprising an organic acid, and either acerola cherry fermentate, or oat extract, or both in an amount and for a period of time sufficient to increase the rate of natural skin desquamation.

It is noted that, unless otherwise stated, all percentages given in this specification and the appended claims refer to percentages by weight.

### DETAILED DESCRIPTION OF THE INVENTION

In accordance with the present invention, a skin, hair, and nail composition is provided and comprises at least one organic acid and either acerola cherry fermentate or oat extract or both. In another aspect, the composition comprises one or more exfoliating agents and either acerola cherry fermentate or oat extract or both to enhance the exfoliation effect of the exfoliating agents.

The hydroxycarboxylic acids can be any of the known hydroxycarboxylic acids but are preferably 2-hydroxycarboxylic acids and related compounds. For convenience, the 2-hydroxycarboxylic acids and related compounds which may be used in accordance with this invention may be classified into three groups, namely (1) 2-hydroxycarboxylic acids, (2) 2-ketocarboxylic adds and esters thereof, and (3) other related compounds. The related compounds may include hydroxycarboxylic acids with the hydroxyl group at any position other than position 2, for example position 3, position 4 or position 5, as well as cyclic hydroxycarboxylic acids (e.g., ascorbic acid and quinic acid), and also may include ketocarboxylic acids and esters thereof. Preferred related compounds include 3-hydroxycarboxylic acids, and 2-ketocarboxylic acids and esters thereof.

### Group 1

The first group comprises organic carboxylic acids in which one hydroxy group is attached to the 2-position carbon atom of the acid. The generic structure of such 2-hydroxycarboxylic acids may be represented as follows:

(Rₐ)(R_{b})C(OH)COOH

where Rₐ and R_{b} may be the same or different and are independently selected from H, F, Cl, Br, alkyl, aralkyl or aryl group of saturated or unsaturated, isomeric or non-isomeric, straight or branched chain or cyclic form, having 1 to 29 carbon atoms, and in addition Rₐ and R_{b} may carry OH, CHO, COOH and alkoxy group having 1 to 9 carbon atoms. 2-Hydroxycarboxylic acids may be present as a free acid or lactone form, or in a salt form with an organic base or an inorganic alkali. 2-Hydroxycarboxylic acids may exist as stereoisomers as D, L, and DL forms when Rₐ and R_{b} are not identical.

Typical alkyl, aralkyl and aryl groups for Rₐ and R_{b} include methyl, ethyl, propyl, isopropyl, butyl, pentyl, octyl, decyl, dodecyl, hexadecyl, benzyl, and phenyl, etc. 2-Hydroxycarboxylic acids of the first group may be further divided into subgroups comprising (1) alkyl hydroxycarboxylic acids, (2) aralkyl and aryl hydroxycarboxylic acids, (3) polyhydroxy-carboxylic acids, and (4) hydroxypolycarboxylic acids. The following are representative 2-hydroxycarboxylic acids in each subgroup.

| **(1) Alkyl Hydroxycarboxylic Acids** | |
|---|---|
| 1. | 2-Hydroxyethanoic acid (Glycolic acid, hydroxyacetic acid) |
| | (H) (H) C (OH) COOH |
| 2. | 2-Hydroxypropanoic acid (Lactic acid) |
| | (CH₃) (H) C (OH) COOH |
| 3. | 2-Methyl 2-hydroxypropanoic acid (Methyllactic acid) |
| | (CH₃) (CH₃) C (OH) COOH |
| 4. | 2-Hydroxybutanoic acid |
| | (C₂H₅) (H) C (OH) COOH |
| 5. | 2-Hydroxypentanoic acid |
| | (C₃H₇) (H) C (OH) COOH |
| 6. | 2-Hydroxyhexanoic acid |
| | (C₄H₉) (H) C (OH) COOH |
| 7. | 2-Hydroxyheptanoic acid |
| | (C₅H₁₁) (H) C (OH) COOH |
| 8. | 2-Hydroxyoctanoic acid |
| | (C₆H₁₃) (H) C (OH) COOH |
| 9. | 2-Hydroxynonanoic acid |
| | (C₇H₁₅) (H) C (OH) COOH |
| 10. | 2-Hydroxydecanoic acid |
| | (C₈H₁₇) (H) C (OH) COOH |
| 11. | 2-Hydroxyundecanoic acid |
| | (C₉H₁₉) (H) C (OH) COOH |
| 12. | 2-Hydroxydodecanoic acid (Alpha hydroxylauric acid) |
| | (C₁₀H₂₁) (H) C (OH) COOH |
| 13. | 2-Hydroxytetradecanoic acid (Alpha hydroxymyristic acid) |
| | (C₁₂H₂₅) (H) C (OH) COOH |
| 14. | 2-Hydroxyhexadecanoic acid (Alpha hydroxypalmitic acid) |
| | (C₁₄H₂₉) (H) C (OH) COOH |
| 15. | 2-Hydroxyoctadecanoic acid (Alpha hydroxystearic acid) |
| | (C₁₆H₃₃) (H) C (OH) COOH |
| 16. | 2-Hydroxyeicosanoic acid (Alpha hydroxyarachidonic acid) |
| | (C₁₈H₃₇) (H) C (OH) COOH |
| 17. | 2-Hydroxytetraeicosanoic acid (Cerebronic acid) |
| | (C₂₂H₄₅) (H) C (OH) COOH |
| 18. | 2-Hydroxytetraeicosenoic acid (Alpha hydroxynervonic acid) |
| | (C₂₂H₄₃) (H) C (OH) COOH |

| **(2) Aralkyl and Aryl 2 -Hydroxycarboxylic Acids** | |
|---|---|
| 1. | 2-Phenyl 2-hydroxyethanoic acid (Mandelic acid) |
| | (C₆H₅) (H) C (OH) COOH |
| 2. | 2,2-Diphenyl 2-hydroxyethanoic acid (Benzilic acid) |
| | (C₆H₅) (C₆H₅) C (OH) COOH |
| 3. | 3-Phenyl 2-hydroxypropanoic acid (Phenyllactic acid) |
| | C₆H₅CH₂) (H) C (OH) COOH |
| 4. | 2-Phenyl 2-methyl 2-hydroxyethanoic acid (Atrolactic acid) |
| | (C₆H₅) (CH₃) C (OH) COOH |
| 5. | 2-(4'-Hydroxyphenyl) 2-hydroxyethanoic acid (4-Hydroxymandelic acid) |
| | (HO-C₆H₄) (H) C (OH) COOH |
| 6. | 2-(4'-Chlorophenyl) 2-hydroxyethanoic acid (4-Chloromandelic acid) |
| | (C1-C₆H₄) (H) C (OH) COOH |
| 7. | 2-(3'-Hydroxy-4'-methoxyphenyl) 2-hydroxyethanoic acid (3-Hydroxy-4-methoxymandelic acid) |
| | (HO-, CH₃O-C₆H₃) (H) C (OH) COOH |
| 8. | 2-(4'-Hydroxy-3'-methoxyphenyl) 2 -hydroxyethanoic acid (4-Hydroxy-3- |
| | methoxymandelic acid) |
| | (HO-, CH₃O-C₆H₃) (H) C (OH) COOH |
| 9. | 3-(2'-Hydroxyphenyl) 2-hydroxypropanoic acid |
| | [3-(2'Hydroxyphenyl) lactic acid] |
| | (HO-C₆H₄-CH₂) (H) C (OH) COOH |
| 10. | 3-(4'-Hydroxyphenyl) 2-hydroxypropanoic acid [3-(4'-Hydroxyphenyl) lactic acid] |
| | (HO-C₆H₄CH₂) (H) C (OH) COOH |
| 11. | 2-(3',4'-Dihydroxyphenyl) 2-hydroxyethanoic acid (3,4-Dihydroxymandelic acid) |
| | (HO-, HO-C₆H₃) (H) C (OH) COOH |

| **(3) Polyhydroxy-Carboxylic Acids** | |
|---|---|
| 1. | 2,3-Dihydroxypropanoic acid (Glyceric acid) |
| | (HOCH₂) (H) C (OH) COOH |
| 2. | 2,3,4-Trihydroxybutanoic acid (Isomers; erythronic acid, threonic acid) |
| | (HOCH₂ HOCH) (H) C (OH) COOH |
| 3. | 2,3,4,5-Tetrahydroxypentanoic acid (Isomers; ribonic acid, arabinoic acid, xylonic acid, lyxonic acid) |
| | (HOCH₂ HOCH HOCH) (H) C (OH) COOH |
| 4, | 2,3,4,5,6-Pntahydroxyhexanoic acid (Isomers; allonic acid, altronic acid, gluconic acid, mannoic acid, gulonic acid, idonic acid, galactonic acid, talonic acid) |
| | (HOCH₂ HOCH HOCH HOCH) (H) C (OH) COOH |
| 5. | 2,3,4,5,6,7-Hexahydroxyheptanoic acid (Isomers; glucoheptonic acid, galactoheptonic acid etc.) |
| | (HOCH₂ HOCH HOCH HOCH HOCH) (H) C (OH) COOH |

| **(4) Hydroxy-Polycarboxylic Acids** | |
|---|---|
| 1. | 2-Hydroxypropane-1,3-dioic acid (Tartronic acid) |
| | (HOOC) (H) C (OH) COOH |
| 2. | 2-Hydroxybutane-1,4-dioic acid (Malic acid) |
| | (HOOC CH₂) (H) C (OH)COOH |
| 3. | 2,3-Dihydroxybutane-1,4-dioic acid (Tartaric acid) |
| | (HOOC HOCH) (H) C (OH) COOH |
| 4. | 2-Hydroxy-2-carboxypentane-1,5-dioic acid (Citric acid) |
| | (HOOC CH₂)₂ C (OH) COOH 2,3,4,5-Tetrahydroxyhexane-1,6-dioic acid (Isomers; saccharic acid, mucic acid etc.) |
| | HOOC (CHOH)₄ COOH |

The 2-hydroxycarboxylic acids may be present in forms other than the acid, such as, for example, salts or lactones. Typical lactone forms that may be used in accordance with this invention include, for example, gluconolactone, galactonolactone, glucuronolactone, galacturonolactone, gulonolactone, ribonolactone, saccharic acid lactone, pantoyllactone, glucoheptonolactone, mannonolactone, and galactoheptonolactone.

### Group 2

The second group, which comprises compounds related to the 2-hydroxycarboxylic acids, includes organic carboxylic acids in which one keto group is attached to position 2 carbon atom of the acid. The generic structure of such 2-ketoacids may be represented as follows:

(R_{c})CO COO(R_{d})

wherein R_{c} and R_{d} can be the same or different and are each selected from H, alkyl, aralkyl or aryl group of saturated or unsaturated, isomeric or non-isomeric, straight or branched chain or cyclic form, having 1 to 29 carbon atoms, and in addition R_{c} may carry F, Cl, Br, I, OH, CHO, COOH and alkoxy group having 1 to 9 carbon atoms. The alpha ketoacids may be present as a free acid or an ester form, or in a salt form with an organic base or an inorganic alkali. The typical alkyl, aralkyl and aryl groups for R_{c} and R_{d} include methyl, ethyl, propyl, 2-propyl, butyl, pentyl, hexyl, octyl, dodecyl, hexadecyl, benzyl and phenyl.

In contract to 2-hydroxycarboxylic acids of the first group compounds, the ester form of 2-ketocarboxylic acids has been found to be therapeutically effective for signs and symptoms of cutaneous aging including intrinsic and extrinsic aging. For example, while methyl 2-hydroxypropanoate and ethyl 2-hydroxypropanoate have minimal effects, methyl 2-ketopropanoate and ethyl 2-ketopropanoate are therapeutically very effective. The real mechanism for such difference is not known. We have speculated that the ester form of the 2-ketocarboxylic acid is chemically and/or biochemically very reactive, and a free 2-ketocarboxylic acid may be released in the skin after penetration through the stratum comeum of the skin. The representative 2-ketocarboxylic acids and their esters of the second group are listed below:

| | |
|---|---|
| 1. | 2-Ketoethanoic acid (Glyoxylic acid) |
| | (H) CO COOH |
| 2. | Methyl 2-ketoethanoate |
| | (H) CO COOCH₃ |
| 3. | 2-Ketopropanoic acid (Pyruvic acid) |
| | CH₃ CO COOH |
| 4. | Methyl 2-ketopropanoate (Methyl pyruvate) |
| | CH₃ CO COOCH₃ |
| 5. | Ethyl 2-ketopropanoate (Ethyl pyruvate) |
| | CH₃ CO COOC₂H₅ |
| 6. | Propyl 2-ketopropanoate (Propyl pyruvate) |
| | CH₃ CO COOC₃H₇ |
| 7. | 2-Phenyl-2-ketoethanoic acid (Benzoylformic acid) |
| | C₆H₅ CO COOH |
| 8. | Methyl 2-phenyl-2 ketoethanoate (Methyl benzoylformate) |
| | C₆H₅ CO COOCH₃ |
| 9. | Ethyl 2-phenyl-2-ketoethanoate (Ethyl benzoylformate) |
| | C₆H₅ CO COOC₂H₅ |
| 10. | 3-Phenyl-2-ketopropanoic acid (Phenylpyruvic acid) |
| | C₆H₅CH₂ CO COOH |
| 11. | Methyl 3-phenyl-2-ketopropanoate (Methyl phenylpyruvate) |
| | C₆H₅CH₂ CO COOCH₃ |
| 12. | Ethyl 3-phenyl-2-ketopropanoate (Ethyl phenylpyruvate) |
| | C6H₆CH₂ CO COOC₂H₅ |
| 13. | 2-ketobutanoic acid |
| | C₂H₅ CO COOH |
| 14. | 2-Ketopentanoic acid |
| | C₃H₇ CO COOH |
| 15. | 2-Ketohexanoic acid |
| | C₄H₉ CO COOH |
| 16. | 2-Ketoheptanoic acid |
| | C₅H₁₁ CO COOH |
| 17. | 2-Ketooctanoic acid |
| | C₆H₁₃ CO COOH |
| 18. | 2-Ketododecanoic acid |
| | C₁₀H₂₁ CO COOH |
| 19. | Methyl 2-ketooctanoate |
| | C₆H₁₄ CO COOCH₃ |

### Group 3

The third group, which also comprises related compounds, includes, among others, hydroxycarboxylic acids where the hydroxy is at a position other than position 2, and cyclic hydroxycarboxylic acids which are useful for topical application to improve signs of aging skin and the cutaneous appendages. The members of this group, which are more conveniently identified by name than by generic structures, include salicylic and other beta-hydroxy carboxylic acids as well as, ascorbic acid, quinic acid, isocitric acid, tropic acid (2-phenyl 3-hydroxypropanoic acid), trethocanic acid, 3-chlorolactic acid, citramalic acid, agaricic acid, alcuritic acid, pantoic add, lactobionic acid and hexulosonic acid.

### Acerola Cherry Fermentate

The acerola cherry is not, in fact, a true cherry but has come to include the cherry-like berries that are produced by any of several shrubs of the *Malpighia* family. These cherry-like berries are also known by other names depending on where they are produced, for instance, West Indian cherry, Barbados cherry, Surinam cherry, and Cereza. The term "acerola cherry" as used in the specification and appended claims is intended to be generic for all of these different berries of the *Malpighia* family.

The acerola cherry fermentate used in the present invention contains only a minor amount of organic acids, particularly carboxylic acids, which includes saturated and unsaturated carboxylic and dicarboxylic acids, hydroxymonocarboxylicic acids, hydroxydicarboxylic acids, and hydroxytricarboxylic acids. In particular, any organic acid or carboxylic acid present in the acerola cherry fermentate is present in an amount no greater than about 5% by weight, preferably no greater than about 3%, and more preferably no greater than about 2% of the acerola cherry fermentate.

The acerola cherry fermentate can be made in any suitable manner to achieve an extract that contains only a minor amount of organic acids, preferably less than about 5% of organic acids. The raw acerola cherry used to make the fermentate may be brown, green, yellow, red or a mixture of two or more, depending upon the availability.

Preferably, the acerola cherry fermentate is obtained from Collaborative Laboratories (NY) and it is believed that the process for making the Acerola cherry fermentate useful in the composition of the present invention is as follows. A cherry extract is prepared by washing the bulk cherries, pitting them and then reducing them to a puree, which is subjected to shearing and extraction with water whereby a filtrate is produced. Thereafter, the filtrate is fermented using bacteria, preferably bacteria from the *lactobacillus* family, to produce a fermentate that is separated from the bulk filtrate. The separated fermentate is the acerola cherry fermentate useful in the present invention.

In the composition according to the present invention, the amount of acerola cherry fermentate to be used can not be absolutely specified because it varies according to the form of the preparation. It is, however, generally used in an amount from about 0.01% to about 50%, more generally from about 0.1% to about 10%. Preferably the acerola cherry fermentate is used in an amount from about 0.5% to about 8%, more preferably from about 1% to about 5% based on the whole weight of the composition.

### Oat Extract

The oat extract is a plant extract derived from oats, *avena sativa.* The oat extract can be incorporated into a number of solvents for ease of use. In the preferred, embodiment, the oat extract is in a 1:1 v/v mixture of 1,3 butylene glycol and water. Thus, in this mixture, the oat extract is present in an amount of about 10% with the butylene glycol and water being present in an amount of about 45%. In this preferred embodiment, the oat extract is obtained from Canamino Inc. under their trade name Ostar™ Arriveen BG.

Surprisingly, the advantageous results achieved by the incorporation of the oat extract are realized even though the oat extract contains no measurable amount of β-glucan. The term "no measurable amount" as used in the specification and claims means that the oat extract contains less than about 1%, preferably less than about 0.1%, and more preferably less than about 0.01% of β-glucan.

To prepare a therapeutic composition in solution form at least one of the aforementioned hydroxycarboxylic acids and either the acerola cherry fermentate or oat extract or both are incorporated into pharmaceutically acceptable vehicles. Desirably, the hydroxycarboxylic acid is selected from the group consisting of lactic acid, malic acid, citric acid, glycolic acid and mixtures thereof.

The concentration of hydroxycarboxylic acid may range from about 0.01 to about 99 percent by weight of the total composition. Preferably, the concentration of hydroxycarboxylic acids ranges from about 0.1% to about 70%, more preferably from about 1% to about 15%, and desirably from about 1% to about 10%.

The concentration of the acerola cherry fermentate ranges from about 0.01 to about 99 percent by weight of the total composition. Preferably, the concentration of acerola cherry fermentate ranges from about 0.05% to about 30%, more preferably from about 0.1% to about 10%.

Desirably, the ratio of organic acid to acerola cherry fermentate ranges from about 1:1 to about 100:1. It is believed that when the ratio of the organic acid to the acerola cherry fermentate is within this range the irritation of acid is reduced and cell renewal rate is incremental. Within this ratio, it is desired to have a composition with a pH between about 3.0 and about 5.0, preferably between about 3.5 and about 4.5, more preferably, between about 3.8 and about 4.2.

The concentration of the oat extract ranges from about 0.01 to about 99 percent by weight of the total composition. Preferably, the concentration of oat extract ranges from about 0.05% to about 30%, more preferably from about 0.1% to about 10%.

Desirably, the ratio of hydroxycarboxylic acid to oat extract ranges from about 1:1 to about 100:1. It is believed that when the ratio of the hydroxycarboxylic acid to the oat extract is within this range the irritation of acid is reduced and cell renewal rate is incremental. Within this ratio, it is desired to have a composition with a pH between about 3.0 and about 5.0, preferably between about 3.5 and about 4.5.

Therapeutic compositions of the present invention may be formulated as a solution, gel, lotion, cream ointment, or other pharmaceutically acceptable form. The compositions of the present invention may also contain various known and conventional cosmetic ingredients so long as they do not detrimentally affect the desired enhancement of skin desquamation. For example, cosmetic ingredients such as alcohols, fats and oils, surfactants, fatty acids, silicones, humectants, moisturizers, viscosity modifiers, emulsifiers, stabilizers, coloring agents, and perfumes or fragrances may be included.

Examples of suitable fatty acids include those chosen from linoleic acid, γ-linolenic acid, homo-γ-linolenic acid, columbinic acid, eicosa-(n-6,9,13)-trienoic acid, arachidonic acid, α-linolenic acid, timnodonic acid, hexaenoic acid and mixtures thereof.

Non-essential fatty acids can also be employed in addition to or in place of essential fatty acids, examples of which are chosen from myristic, palmitic, stearic and isostearic acids, and mixtures thereof.

The composition according to the invention also comprises a cosmetically acceptable aqueous or non-aqueous vehicle to act as a diluant, dispersant or carrier to facilitate distribution when the composition is applied to the skin, hair and/or nails.

Vehicles other than water can include liquid or solid emollients, solvents, humectants, thickeners and powders. Examples of each of these types of vehicle, which can be used single or as mixtures of one or more vehicles, are as follows:

Emollients, such as stearyl alcohol, glyceryl monoricinoleate, glyceryl monostearate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eiconsanyl alcohol, behenyl alcohol, cetyl palmitate, volatile or non-volatile silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, tallow, lard, olive oil, palm kernel oil, rapeseed oil, safflower seed oil, evening primrose oil, soybean oil, sunflower seed oil, passion flower oil, avocado oil, olive oil, sesame seed oil, coconut oil, arachis oil, castor oil, acetylated lanolin alcohols, petroleum jelly, mineral oil, butyl myristate, isostearic acid, palmitatic acid, isopropyl linoleate, lauryl lactate, myristyl lactate, decyl oleate, myristyl myristate;

Propellants, such as air, propane, butane, isobutane, dimethyl ether, carbon dioxide, nitrous oxide;

Solvents such as ethyl alcohol, methylene chloride, isopropanol, acetone, squalane, squalene, ethylene glycol monethyl ether, diethylene glycol monobutyl ether, diethylene glycol monoethyl ether, polyethylene glycol, dimethyl sulphoxide, dimethyl formamide, butylene glycol, tetrahydrofuran;

Powders such as chalk, talc, fullers earth, kaolin, starch, gums, colloidal silica sodium polyacrylate, tetra alkyl and/or trialkyl aryl ammonium smectites, chemically modified magnesium aluminum silicate, organically modified montmorillonite clay, hydrated aluminum silicate, fumed silica, carboxyvinyl polymer, sodium carboxymethyl cellulose, ethylene glycol monostearate.

The cosmetically acceptable vehicle will usually form from 10 to 99.9%, preferably from 50 to 99% by weight of the emulsion, and can, in the absence of other cosmetic adjuncts, form the balance of the composition.

Although the composition according to the invention can be aqueous or non-aqueous, a particularly convenient form is an emulsion, in which case an oil or oily material will normally be present, together with an emulsifier to provide a water-in-oil emulsion, an oil-in-water emulsion, or a complex emulsion, depending largely on the average hydrophilic-lipophilic balance (HLB) of the emulsifier employed.

The composition can optionally comprise one or more oils or other materials having the properties of an oil. Examples of suitable oils include mineral oil and vegetable oils, and oil materials, such as those already proposed herein as emollients. Other oils or oily materials include silicone oils, both volatile and non-volatile, such as polydimethyl siloxanes. The oil or oily material, when present for the purposes for forming an emulsion, will normally form up to 90%, preferably from 10 to 80% by volume of the composition.

The composition can also optionally comprise one or more emulsifiers the choice of which will normally determine whether a water-in-oil or and oil-in-water emulsion is formed. When a water-in-oil emulsion is required, the chosen emulsifier or emulsifiers should normally have an average HLB value of from 1 to 6. When an oil-in-water emulsion is required, a chosen emulsifier or emulsifiers should have an average HLB value of >6.

Examples of suitable emulsifiers are set forth below in Table 1 in which the chemical name of the emulsifiers is given together with an example of a trade name as commercially available, and the average HLB value.

**Table 1**

| **Chemical Name of Emulsifier** | **Trade Name** | **HLB Value** |
|---|---|---|
| Sorbitan trioleate | Arlacel 85 | 1.8 |
| Sorbitan tristearate | Span 65 | 2.1 |
| Glycerol monoleate | Aldo MD | 2.7 |
| Glycerol monostearate | Atmul 84S | 2.8 |
| Glycerol monolaurate | Aldo MC | 3.3 |
| Sorbitan sesquioleate | Arlacel 83 | 3.7 |
| Sorbitan monooleate | Arlacel 80 | 4.3 |
| Sorbitan monostearate | Arlacel 60 | 4.7 |
| Poloxyethylene (2) stearyl ether | Brij 72 | 4.9 |
| Poloxyethylene sorbitolbeeswax derivative | G-1702 | 5 |
| PEG 200 dilaurate | Emerest 2622 | 6.3 |
| Sorbitan monopalmitate | Arlacel 40 | 6.7 |
| Polyoxyethylene (3.5) nonyl phenol | Emulgen 903 | 7.8 |
| PEG 200 monostearate | Tegester PEG 200 MS | 8.5 |
| Sorbitan monolaurate | Arlacel 200 400-DO | 8.6 - 8.8 |
| Polyoxyethylene (5) monostearate | Ethofat 60-16 | 9.0 |
| Polyoxyethylene (4) sorbitan monostearate | Tween 61 | 9.6 |
| Polyoxyethylene (4) lauryl ether | Brij 30 | 9.7 |
| Polyoxyethylene (5) sorbitan monooleate | Tween 81 | 10.0 |
| PEG 300 monooleate | Neutronyx 834 | 10.4 |
| Polyoxyetheylene (20) sorbitan tristearate | Tween 65 | 10.5 |
| Polyoxyetheylene (20) sorbitan trioleate | Tween 85 | 11.0 |
| Polyoxyetheylene (8) monostearate | Myrj 45 | 11.1 |
| PEG 400 monooleate | Emerest 2646 | 11.7 |
| PEG 400 monostearate | Tegester PEG 400 | 11.9 |
| Polyoxyetheylene 10 monooleate | Ethofat 0/20 | 12.2 |
| Polyoxyetheylene (10) stearyl ether | Brij 76 | 12.4 |
| Polyoxyetheylene (10) cetyl ether | Brij 56 | 12.9 |
| Polyoxyetheylene (9.3) octyl phenol | Triton X-100 | 13.0 |
| Polyoxyetheylene (4) sorbitan monolaurate | Tween 21 | 13.3 |
| PEG 600 monooleate | Emerest 2660 | 13.7 |
| PEG 1000 dilaurate | Kessco | 13.9 |
| Polyoxyetheylene sorbitol lanolin derivative | G-1441 | 14.0 |
| Polyoxyetheylene (12) lauryl ether | Ethosperse LA-12 | 14.4 |
| PEG 1500 dioleate | Pegosperse 1500 | 14.6 |
| Polyoxyetheylene (14) laurate | Arosurf HFL-714 | 14.8 |
| Polyoxyetheylene (20) sorbitan monostearate | Tween | 14.9 |
| Polyoxyetheylene 20 sorbitan monooleate | Tween 80 | 15.0 |
| Polyoxyetheylene (20) stearyl ether | Brij 78 | 15.3 |
| Polyoxyetheylene (20) sorbitan monopalmitate | Tween 40 | 15.6 |
| Polyoxyetheylene (20) cetyl ether | Brij 58 | 15.7 |
| Polyoxyetheylene (25) oxypropylene monostearate | G-2162 | 16.0 |
| Polyoxyetheylene (20) sorbitol monolaurate | Tween 20 | 16.7 |
| Polyoxyetheylene (23) lauryl ether | Brij 35 | 16.9 |
| Polyoxyetheylene (50) monostearate | Myrj 53 | 17.9 |
| PEG 4000 monostearate | Pegosperse 4000 MS | 18.7 |

The foregoing list of emulsifiers is not intended to be limiting and merely exemplifies selected emulsifiers that are suitable for use in accordance with the invention. It is to be understood that two or more emulsifiers can be employed if desired.

The amount of emulsifier or mixtures thereof, to be incorporated in the composition of the invention, when appropriate is from 1 to 50%, preferably from 2 to 20% and most preferably from 2 to 10% by weight of the composition.

Although the composition of the invention can be anhydrous, it can also comprise water, usually up to 98%, preferably from 5 to 80% by volume.

The composition of the invention can also optionally comprise a high molecular weight silicone surfactant that can also act as an emulsifier, in place of or in addition to the optional emulsifiers already mentioned. One example of a suitable silicone surfactant is a high molecular weight polymer of dimethyl polysiloxane with polyoxyethylene and/or polyoxypropylene side chains having a molecular weight of from 10,000 to 50,000. The dimethyl polysiloxane polymer is may be provided as a dispersion in a volatile siloxane, the dispersion comprising, for example, from 1 to 20% by volume of the polymer and from 80 to 99% by volume of the volatile siloxane. Ideally, the dispersion consists of a 10% by volume of the polymer dispersed in the volatile siloxane. Examples of the volatile siloxanes in which the polysiloxane polymer can be dispersed include polydimethyl siloxane (pentamer and/or hexamer).

A preferred silicone surfactant is cyclomethicone and dimethicone copolyol, such as DC 3225C Formulation Aid available from DOW CORNING. Another is laurylmethicone copolyol, such as DC Q2--5200, also available from Dow Coming.

The amount of silicone surfactant, when present in the composition will normally be from 0.1% to 25%, preferably from 0.5 to 15% by weight of the emulsion.

Examples of other conventional adjuncts which can optionally be employed include preservatives, such as para-hydroxy benzoate esters; antioxidants, such as α-tocopherol, humectants, such as glycerol, sorbitol, 2-pyrrolidone-5carboxylate, dibutylphthalate, gelatin, polyethylene, glycol, preferably PEG 200-600; buffers, such as lactic acid together with-a base such as triethanolamine or sodium hydroxide; surfactants, such as glycerol ethers; waxes, such as beeswax, ozokerite wax, paraffin wax, plant extracts, such as aloe vera, cornflower, witch hazel, elderflower, cucumber; thickeners; activity enhancers; colorants; perfumes; and sunscreen materials such as ultrafine titanium dioxide and organic sunscreens such as p-aminobenzoic acid and esters thereof, ethylhexyl p-methoxycinnamate, 2-ethoxyethyl p-methoxycinnamate and butyl methoxydibenzoylmethane; and skin benefit agents, such as retinoic acid, retinol, retinol esters; anti-inflammatory agents, such as salicylic acid; skin whiteners, such as arbutin and mixtures thereof.

In accordance with one aspect of the present invention, the rate of natural skin desquamation may increased by topical application to the skin of the above compositions. In this regard, the present invention encompasses a method of enhancing the rate of natural skin desquamation comprising topically applying to the skin a composition comprising a hydroxycarboxylic acid and either or both acerola cherry fermentate and oat extract in an amount and for a period of time sufficient to increase the rate of natural skin desquamation. Preferably, the composition is as described above.

Generally, the topical application is on at least a daily basis and may be applied for any suitable period of time. Within a few days, a user may notice improvement in skin texture and smoothness.

The present invention also includes a method of enhancing a composition used for skin desquamation and including a hydroxycarboxylic acid wherein the method includes incorporating an enhancing effective amount of acerola cherry extract and/or oat extract

The following are illustrative examples of formulations and compositions according to this invention. Although the examples use only selected compounds and formulations, it should be understood that the following examples are illustrative and not limited. Thus, any of the aforementioned hydroxycarboxylic acids and related compounds may be substituted according to the teachings of this invention in the following examples.

Table 2 sets forth a preferred embodiment of a lotion according to the present invention.

**TABLE 2**

| **INGREDIENT** | **AMOUNT (wt%)** |
|---|---|
| Lactic Acid (88% active) | 4.50 |
| Citric, Malic, and Lactic acid mixture (30-40% active) | 3.00 |
| Acerola Cherry Fermentate (1.8-3% active acid) | 3.00 |
| Glyceryl monostearate PEG100 Stearate | 3.00 |
| Sodium Hydroxide | 2.30 |
| Glycerin | 2.00 |
| Butylene Glycol | 2.00 |
| Dimethicone | 2.00 |
| Stearic Acid | 2.00 |
| Phenonip | 0.75 |
| Sorbitan Stearate | 0.50 |
| Magnesium Aluminum Silicate | 0.40 |
| Cetyl Alcohol | 0.25 |
| Xanthan Gum | 0.20 |
| Methylparaben | 0.15 |
| Water and adjuncts | qs to 100.00 |
| **TOTAL** | **100.00** |

Adjuncts include but are not limited to preservatives such as methyl paraben, phenonip, gums such as magnesium aluminum silicate, hydroxyethyl cellulose, and xanthan gum, humectants such as panthenol, glycerin and butylene glycol, waxes such as sorbitan stearate and glyceryl monostearate and PEG 100 stearate, stearic acid, cetyl alcohol, skin conditioning agents such as alkyl benzoates, dimethicone, lipids, hyaluronic acid and salts thereof, green tea, ginko biloba, polyglyceryl methacrylate, propylene glycol.

Table 3 presents a gel-type formula falling within the scope of the present invention with the amounts provided being expressed as weight percent.

**TABLE 3**

| **INGREDIENT** | **AMOUNT (wt%)** |
|---|---|
| Lactic Acid (88% active acid) | 2.25 |
| Citric, Malic, and Lactic acid mixture (30-40% active) | 3.00 |
| Acerola Cherry Fermentate (1.8-3% active acid) | 3.00 |
| PEG-20 | 5.00 |
| Glycerin | 4.00 |
| Polyglyceryl methacrylate, Propylene Glycol | 4.00 |
| Butylene Glycol | 3.00 |
| Sodium Hydroxide | 1.40 |
| Hydroxyethyl cellulose | 0.40 |
| Xanthan gum | 0.40 |
| Saccharide isomerate | 0.30 |
| Water and adjuncts | qs to 100.00 |
| **TOTAL** | **100.00** |

In order to determine whether compositions containing organic acids and acerola cherry fermentate were therapeutically effective in enhancing the natural rate of skin desquamation the following tests were conducted. The skin cell renewal, irritation level and therapeutic index were measured according to the procedure described in *Soap*/*Cosmetics*/*Chemical Specialties for September 1993* at pp.55-59.

**TABLE 4**

| **Formula** | **AHA*%** | **Acerola Fermentate %** | **% Cell Renewal** | **Irritation Level** | **Therapeutic Index** |
|---|---|---|---|---|---|
| A | 3.0 | 0 | 12.2 | 11.4 | 10.7 |
| B | 3.0 | 3.0 | 19.8 | 15.6 | 11.5 |
| C | 3.0 | 3.0 | 21.4 | 16.8 | 12.7 |
| D | 5.0 | 3.0 | 19.8 | 14.2 | 14.0 |
| E | 5.0 | 3.0 | 18.9 | 14.1 | 13.4 |
| F | 5.0 | 3.0 | 22.6 | 13.1 | 17.3 |

| | | | | | |
|---|---|---|---|---|---|
| *AHA refers to the total amount of active hydroxycarboxylic acid selected from glycolic acid, malic acid, citric acid, lactic acid and mixtures thereof. | | | | | |

Formulas A-E were made according to the formula set forth in Table 2 with the amounts of water and alpha-hydroxy acid being varied so that the alpha-hydroxy acid was present in the amounts shown in Formulas A-E, above. Formula F was made according to the formula set forth in Table 3 with the amount of water and alpha-hydroxy acid varied to the level shown for Formula F. The results show that the addition of the acerola cherry fermentate surprisingly and unexpectedly significantly increased cell renewal rate while only slightly increasing irritation.

Table 4 sets forth a preferred embodiment of one aspect of the present invention.

**TABLE 4**

| INGREDIENT | AMOUNT (wt%) |
|---|---|
| D-I-Water | 70.54 |
| PEG-20 (Polyethylene Glycol) | 5.00 |
| Lactic Acid (88%) | 4.50 |
| Glycerin | 4.00 |
| Butylene Glycol | 3.00 |
| Lactic Acid/Citric Acid/Malic Acid/Green Tea Extract | 3.00 |
| Acerola Extract Fermentate | 3.00 |
| Sodium PCA | 2.00 |
| NaOH (50%) | 1.40 |
| Oat Extract (Arriveen BG) | 1.00 |
| Panthenol | 0.50 |
| Thickeners, extracts, preservatives, skin conditioners | 2.06 |

Table 5 presents several examples of formulas falling within the scope of the present invention with the amounts provided being expressed as weight percent.

**TABLE 5**

| **Ingredient** | **Example 1** | **Example 2** | **Example 3** | **Example 4** | **Example 5** | **Example 6** |
|---|---|---|---|---|---|---|
| D-I-Water | 70 | 70 | 70 | 70 | 70 | 70 |
| AHA (includes a mixture of one or more of lactic acid, citric acid, malic acid and/or glycolic acid) | 3 | 3 | 5 | 5 | 10 | 10 |
| Oat Extract (Arriveen BG) | 1 | 3 | 1 | 2 | 1 | 5 |
| Skin lotion ingredients including thickeners, extracts, preservatives, skin conditioners | balance | balance | Balance | balance | balance | balance |

In order to determine whether compositions containing hydroxycarboxylic acids and oat extract were therapeutically effective in enhancing the natural rate of skin desquamation the following tests were conducted. The skin cell renewal, irritation level and therapeutic index were measured according to the procedure described in *Soap*/*Cosmetics*/*Chemical Specialties for September 1993* at pp.55-59.

**TABLE 6**

| **Formula** | **Total % AHA*** | **% Panthenol** | **% Oat Extract** | **% Cell Renewal** | **Irritation Level** | **Therapeutic Index** |
|---|---|---|---|---|---|---|
| A | 3.0 | 2.0 | - | 12.2 | 11.4 | 10.7 |
| B | 3.0 | 2.0 | - | 19.2 | 13.4 | 14.0 |
| C | 3.0 | 2.0 | - | 19.8 | 15.6 | 11.5 |
| D | 3.0 | 0.5 | - | 21.4 | 16.8 | 12.7 |
| E | 5.0 | 2.0 | - | 19.8 | 14.2 | 14.0 |
| F | 5.0 | 0.5 | - | 18.9 | 14.1 | 13.4 |
| H | 5.0 | 0.5 | 1.0 | 25.6 | 14.5 | 18.0 |
| I | 8.0 | 0.1 | 1.0 | 24.4 | 12.2 | 20.0 |
| J | 5.0 | .05 | 1.0 | 26.6 | 11.2 | 23.8 |
| K | 5.0 | .05 | 1.0 | 28.0 | 12.1 | 23.1 |
| AHA refers to the total amount of hydroxycarboxylic acid selected from glycolic acid, malic acid, citric acid, lactic acid and mixtures thereof. | | | | | | |

The results show that the addition of the oat extract surprisingly and unexpectedly increased cell renewal rate while maintaining or reducing irritation to levels below that when no oat extract is incorporated.

## Claims

1. A cosmetic composition comprising organic acid and a constituent selected from acerola cherry fermentate, oat extract, or mixtures thereof, wherein the oat extract contains less than 1% by weight β-glucan, and wherein the organic acid is a hydroxycarboxylic acid represented by a generic structure of:
RₐR_{b}C(OH)COOH
wherein Rₐ and R_{b} may be the same or different and are independently selected from H, F, Cl, Br alkyl, aralkyl or aryl group of saturated or unsaturated straight or branched chain or cyclic form having 1 to 29 carbon atoms, and in addition Rₐ and R_{b} can be substituted by OH, CHO, COOH and/or alkoxy group having 1 to 9 carbon atoms, said hydroxycarboxylic acid may be present as a free acid or in lactone form, or in a salt form with an organic base or an inorganic alkali, and as stereoisomers as D, L, and/or DL forms when Rₐ and R_{b} are not identical.

2. The composition according to claim 1 wherein the hydroxycarboxylic acid is glycolic, malic, citric or lactic acid, salts thereof or mixtures thereof.

3. The composition according to any one of the preceding claims wherein each of the organic acid, acerola cherry fermentate, and oat extract are present.

4. The composition according to any one of the preceding claims wherein the acerola cherry fermentate contains less than 5% by weight organic acid.

5. The composition according to any one of the preceding claims further comprising a topically acceptable vehicle.

6. The composition according to any one of the preceding claims, wherein the pH is from about 3.5 to 4.5.

7. Use of a composition to improve the appearance of skin comprising applying thereto a composition as defined in any of the preceding claims, which use excludes methods for therapeutic treatment.

8. Use of a composition as defined in any one of claims 1 to 6 in the manufacture of a composition for topical application to the skin.

## Patentansprüche

1. Kosmetische Zusammensetzung, umfassend eine organische Säure und einen Bestandteil, ausgewählt aus Acerolakirschenfermentat, Haferextrakt oder Gemischen davon, wobei der Haferextrakt weniger als 1 Gew.-% β-Glucan enthält und wobei die organische Säure eine Hydroxycarbonsäure ist, die durch die folgende allgemeine Formel wiedergegeben wird:
RₐR_{b}C(OH)COOH
wobei Rₐ und R_{b} gleich oder verschieden sein können und unabhängig ausgewählt werden aus H, F, Cl, Br, einer Alkyl-, Aralkyl- oder Arylgruppe mit einer gesättigten oder ungesättigten geraden oder verzweigten Kette oder cyclischen Form mit 1 bis 29 Kohlenstoffatomen, und außerdem Rₐ und R_{b} durch OH, CHO, COOH und/oder eine Alkoxygruppe mit 1 bis 9 Kohlenstoffatomen substituiert sein können, wobei die Hydroxycarbonsäure als freie Säure oder in Lactonform oder in Form eines Salzes mit einer organischen Base oder einem anorganischen Alkali, und, wenn Rₐ und R_{b} nicht identisch sind, als Stereoisomere als D-, L- undloder DL-Formen vorliegen kann.

2. Zusammensetzung nach Anspruch 1, wobei die Hydroxycarbonsäure Glycolsäure, Äpfelsäure, Citronensäure oder Milchsäure, Salze davon oder Gemische davon ist.

3. Zusammensetzung nach einem der vorangehenden Ansprüche, wobei von der organischen Säure, dem Acerolakirschenfermentat und dem Haferextrakt alle vorhanden sind.

4. Zusammensetzung nach einem der vorangehenden Ansprüche, wobei das Acerolakirschenfermental weniger als 5 Gew.-% organische Säure enthält.

5. Zusammensetzung nach einem der vorangehenden Ansprüche, außerdem umfassend ein topisch annehmbares Vehikel.

6. Zusammensetzung nach einem der vorangehenden Ansprüche, wobei der pH ungefähr 3,5 bis 4,5 beträgt.

7. Verwendung einer Zusammensetzung zum Verbessern des Aussehens der Haut, umfassend das Auftragen einer Zusammensetzung, wie sie in einem der vorangehenden Ansprüche definiert ist, darauf, wobei die Verwendung Verfahren für eine therapeutische Behandlung ausschließt

8. Verwendung einer Zusammensetzung, wie sie in einem der Ansprüche 1 bis 6 definiert ist, bei der Herstellung einer Zusammensetzung für eine topische Anwendung auf die Haut.

## Revendications

1. Composition cosmétique comprenant de l'acide organique et un constituant choisi parmi un fermentat de cerise des Antilles, de l'extrait d'avoine ou des mélanges de ceux-ci, dans laquelle l'extrait d'avoine contient moins de 1% en poids de β-glucane et dans laquelle l'acide organique est un acide hydroxycarboxylique représenté par la structure générale :
RₐR_{b}C(OH)COOH
où Rₐ et R_{b} peuvent être identiques ou différents et sont choisis indépendamment parmi H, F, Cl, Br, un groupe alkyle, aralkyle ou aryle de forme saturée ou insaturée, à chaîne droite ou ramifiée ou cyclique, ayant de 1 à 29 atomes de carbone, et Rₐ et R_{b} peuvent être substitués en plus par un groupe OH, CHO, COOH et/ou alcoxy ayant de 1 à 9 atomes de carbone, ledit acide hydroxycarboxylique pouvant être présent sous la forme d'acide libre ou de lactone ou bien sous la forme d'un sel avec une base organique ou un alcali inorganique, et sous la forme de stéréoisomères de type D, L et/ou DL lorsque Rₐ et R_{b} ne sont pas identiques.

2. Composition selon la revendication 1, dans laquelle l'acide hydroxycarboxylique est l'acide glycolique, malique, citrique ou lactique, des sels de ceux-ci ou des mélanges de ceux-ci.

3. Composition selon l'une quelconque des revendications précédentes, dans laquelle de l'acide organique, du fermentat de cerise des Antilles et de l'extrait d'avoine sont tous les trois présents.

4. Composition selon l'une quelconque des revendications précédentes, dans laquelle le fermentat de cerise des Antilles contient moins de 5% en poids d'acide organique.

5. Composition selon l'une quelconque des revendications précédentes, comprenant en plus un véhicule acceptable pour un usage topique.

6. Composition selon l'une quelconque des revendications précédentes, dont le pH est de 3,5 à 4,5 environ.

7. Utilisation d'une composition pour améliorer l'aspect de la peau, comprenant l'application sur cette dernière d'une composition telle que définie dans l'une quelconque des revendications précédentes, laquelle utilisation exclut des méthodes de traitement thérapeutique.

8. Utilisation d'une composition telle que définie dans l'une quelconque des revendications 1 à 6 pour la fabrication une composition destinée à une application topique sur la peau.
